# EUROPEAN PATENT APPLICATION

(11) **EP 0 555 681 A1**
(43) Date of publication of application: **18.08.1993**
(21) Application number: 93101088.8
(22) Date of filing: 25.01.1993
(51) Int. Cl.: A61K 31/195, A61K 31/40, A61K 31/44

(54) **Use of a combination of potassium channel activators and sulfhydryl containing compounds for the manufacture of a medicament for the treatment of ischemia and myocarolial infarct**

(30) Priority: 13.02.1992 US 835659
(71) Applicant: E.R. SQUIBB & SONS, INC., Princeton New Jersey 08543-4000 (US)
(72) Inventor: Grover, Gary J., Stockton, NJ 08559 (US); Sargent, Carol A., Cranbury, NJ 08512 (US)
(74) Representative: Josif, Albert, Dr.-Ing.

(57) **Abstract**

A method for treating ischemia in mammalian species which includes administering a combination of a potassium channel opener such as cromakalim and a compound having a sulfhydryl moiety such as the angiotensin converting enzyme inhibitor zofenopril.

## Description

The present invention relates to a combination of a potassium channel activator and a sulfhydryl containing compound and to a method for treating ischemia and/or reducing infarct size in mammalian species by administering such combination.

There are several different classes of compounds which may have anti-ischemic properties. The mechanism of action of many of these compounds is not completely understood. Some of these compounds may act at different molecular sites, but ultimately may work via similar physiological mechanisms.

Potassium channel activators (PCA), are a relatively new class of compounds having anti-ischemic properties, Cook, N.S., "The Pharmacology of Potassium Channels and their Therapeutic Potential," TIPS 9:21, 1988. Cook indicates "that the cellular site of action of a number of drugs used therapeutically seems to involve the modulation of membrane K+ channels" and that "opening of K+ channels by a new class of drugs appears to underlie their relaxation of a variety of smooth muscles" because of their ability to hyperpolarize and thus relax these cells.

Potassium channel activators are thought to be effective in myocardial ischemia (Grover, G. et al., "Pharmacologic Profile of cromakalim in the Treatment of Myocardial Ischemia in Isolated Rat Hearts and Anesthetized Dogs", J. Cardiovascular Pharmacology 16:853-864, 1990). More particularly, one class of potassium channel openers are referred to as ATP-sensitive potassium channel openers and have previously been shown to have direct protective effects in ischemic myocardial tissue (Grover, G. et al., Cardiovasc Drug Ther 4:465, 1990). This protective effect is completely abolished by the potassium channel blockers glyburide and sodium 5-hydroxydecanoate which have been shown to be selective for ATP-sensitive potassium channels in the heart (Fosset, M. et al., J Biol Chem 263:7933, 1988). Both glyburide and sodium 5-hydroxydecanoate appear to be ineffective in abolishing the cardioprotective actions of classical anti-ischemic compounds such as calcium antagonists, sodium blockers and calmodulin inhibitors (Sargent, C. et al., J Pharmacol Exp Ther). However, little is presently known about the gating mechanism responsible for the cardioprotective effects of potassium channel openers.

While certain sulfhydryl-containing compounds such as angiotensin converting enzyme (ACE) inhibitors are known as cardiovascular agents, activity of such compounds on ATP-sensitive potassium channels has not been heretofore reported.

It has now been found that the combination of a potassium channel opener and a pharmaceutically acceptable compound having a sulfhydryl moiety is a unique concept in treating ischemia. Specifically, it has been found that the combination specified above has anti-ischemic effects which are substantially greater than what would be expected if the effects of the combination were merely additive.

Figures 1a and 1b show the effect of 1 µM glyburide (G), 100 µM sodium 5-hydroxydecanoate (HD), and 100 µM zofenopril (Z) on end diastolic pressure (EDP) and cumulative LDH release at 30 minutes into reperfusion following 25 minutes of global ischemia in isolated rat hearts.

Figures 2a and 2b show the effect of 1 µM glyburide (G) or 400 µM zofenopril-SH (Z-SH) on end diastolic pressure (EDP) and cumulative LDH release at 30 minutes into reperfusion following 25 minutes of global ischemia in isolated rat hearts. Zofenopril-SH was either given into the oxygenated Krebs buffer reservoir or was given directly (D) into the aortic root via an extracorporeal pump.

Figures 3a and 3b show the effect of 1 µM glyburide (GLY) and 100 or 300 µM N-acetylcysteine (NAC) on end diastolic pressure (EDP) and cumulative LDH release at 30 minutes into reperfusion following 35 minutes of global ischemia in isolated rat hearts.

Figures 4a and 4b show the effect of penicillamine (P) on reperfusion contractile function and cumulative LDH release.

Listed below are definitions of various terms used to describe the instant invention. These definitions apply to the terms as they are used throughout the specification (unless they are otherwise limited in specific instances) either individually or as part of a larger group.

The term "alkyl" refers to straight or branched chain saturated hydrocarbon radicals having up to eight carbons, preferably from one to five carbons. Similarly, the terms "alkoxy" and "alkylthio" refer to such alkyl groups attached to an oxygen or sulfur respectively.

The term "alkenyl" refers to straight or branched chain hydrocarbon radicals having from two to eight carbons and one double bond, preferably three to five carbons. The term "alkynyl" refers to straight or branched chain hydrocarbon radicals having from two to eight carbons and one triple bond, preferably three to five carbons.

The term "cycloalkyl" refers to saturated carbocyclic rings of 3 to 7 carbon atoms with cyclopropyl, cyclopentyl and cyclohexyl being most preferred.

The term "halo" or "halogen" refers to chloro, bromo, iodo and fluoro.

The term "halo substituted alkyl" refers to such alkyl groups described above in which one or more hydrogens have been replaced by chloro, bromo or fluoro groups such as trifluoromethyl, pentafluoroethyl, 2,2,2-trichloroethyl, chloromethyl, bromomethyl, etc., trifluoromethyl being preferred.

The term "aryl" refers to phenyl, 1-naphthyl, 2-naphthyl or mono substituted phenyl, 1-naphthyl, 2-naphthyl wherein said substituent is alkyl of 1 to 4 carbons, alkylthio of 1 to 4 carbons, alkoxy of 1 to 4 carbons, halo, nitro, cyano, hydroxy, amino, -NH-alkyl wherein alkyl is of 1 to 4 carbons, -N(alkyl)₂ wherein alkyl is of 1 to 4 carbons, -N(Rₐ)CORₐ', -N(Rₐ)CO-haloalkyl, -N(Rₐ)CO-amino, -N(Rₐ)CO-substituted amino, -CORₐ, -COORₐ (wherein Rₐ and Rₐ' are independently selected from hydrogen, alkyl, haloalkyl, aryl or arylalkyl), -CF₃, -OCHF₂, 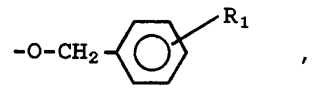 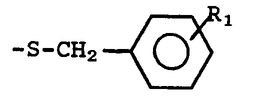
(wherein R₁ is hydrogen, alkyl of 1 to 4 carbons, alkoxy of 1 to 4 carbons, alkylthio of 1 to 4 carbons, halo, hydroxy or -CF₃), -O-CH₂-cycloalkyl, or -S-CH₂-cycloalkyl, and di-substituted phenyl, 1-naphthyl, 2-naphthyl wherein said substituents are selected from methyl, methoxy, methylthio, halo, -CF₃, nitro, amino, and -OCHF₂.

Preferred aryl groups include unsubstituted phenyl and monosubstituted phenyl wherein the substituents are selected from nitro, halo, -CF₃, alkyl, cyano or methoxy.

The term "heterocyclo" refers to fully saturated or unsaturated rings of 5 or 6 atoms containing one or two O and S atoms and/or one to four N atoms provided that the total number of hetero atoms in the ring is 4 or less. The hetero ring is attached by way of an available carbon atom. Preferred monocyclic hetero groups include 2- and 3-thienyl, 2- and 3-furyl, 2-, 3- and 4-pyridyl, and imidazolyl. The term hetero also includes bicyclic rings wherein the five or six membered ring containing O, S and N atoms as defined above is fused to a benzene ring and the bicyclic ring is attached by way of an available carbon atom. Preferred bicyclic hetero groups include 4, 5, 6 or 7-indolyl, 4,5, 6 or 7-isoindolyl, 5, 6, 7 or 8-quinolinyl, 5, 6, 7 or 8-isoquinolinyl, 4, 5, 6 or 7-benzothiazolyl, 4, 5, 6 or 7-benzoxazolyl, 4, 5, 6 or 7-benzimidazolyl, 4, 5, 6 or 7-benzoxadiazolyl, and 4, 5, 6 or 7-benzofuranzanyl.

The term heterocyclo also includes such monocyclic and bicyclic rings wherein an available carbon atom is substituted with a lower alkyl of 1 to 4 carbons, lower alkylthio of 1 to 4 carbons, lower alkoxy of 1 to 4 carbons, halo, nitro, keto, cyano, hydroxy, amino, -NH-alkyl wherien alkyl is of 1 to 4 carbons, -N(alkyl)₂ wherein alkyl is of 1 to 4 carbons, -CF₃, or -OCHF₂ or such monocyclic and bicyclic rings wherein two or three available carbons have substituents selected from methyl, methoxy, methylthio, halo, -CF₃, nitro, hydroxy, amino and -OCHF₂.

The term "substituted amino" refers to a group of the formula -NZ₁Z₂ wherein Z₁ is hydrogen, alkyl, cycloalkyl, aryl, arylalkyl, cycloalkylalkyl and Z₂ is alkyl, cycloalkyl, aryl, arylalkyl, cycloalkylalkyl or Z₁ and Z₂ taken together with the nitrogen atom to which they are attached are 1-pyrrolidinyl, 1-piperidinyl, 1-azepinyl, 4-morpholinyl, 4-thiamorpholinyl, 1-piperazinyl, 4-alkyl-1-piperazinyl, 4-arylalkyl-1-piperazinyl, 4-diarylalkyl-1-piperazinyl, 1-pyrrolidinyl, 1-piperidinyl, or 1-azepinyl substituted with alkyl, alkoxy, alkylthio, halo, trifluoromethyl or hydroxy.

The term "ischemia" refers to a reduction in blood flow such as cerebral ischemia, myocardial ischemia, ischemia in the limbs, and the like, such as caused by intermittent claudication, shock, trauma, hemorrhage, thromboses, clots and the like.

The terms "potassium channel activators" and "potassium channel openers" are used herein interchangeably and include any compound which activates potassium channels. Preferred are those which activate ATP sensitive potassium channels.

Examples of potassium channel activator drugs or compounds include (+)-2-cyano-1-(4-pyridyl)-3-(1,2,2-trimethylpropyl)guanidine (hereinafter referred to as pinacidil) and (±)-6-cyano-3,4-dihydro-2,2-dimethyl-trans-4-(2-oxo-1-pyrrolidyl)-2H-benzo[6]-pyran-3-ol (hereinafter referred to as cromakalim or BRL 34915).

Other examples of compounds which are potassium channel activators include N-(2-hydroxyethyl)nicotinamide nitrate ester (hereinafter referred to as nicorandil) and 6-amino-1,2-dihydro-1-hydroxy-2-imino-4-piperidino-pyrimidine (hereinafter referred to as minoxidil).

Aryl cyanoguanidine potassium channel activators disclosed in U.S. Patent No. 5,011,837, issued April 30, 1991, the disclosure of which is incorporated by reference herein, may be used in accordance with this invention. The aryl cyanoguanidine compounds have the general formula 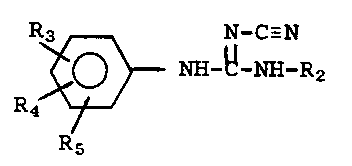
and its possible tautomers 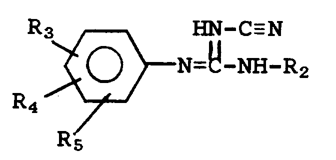
and 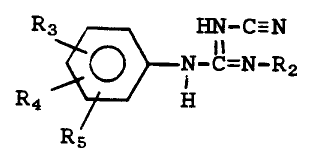
including pharmaceutically acceptable salts wherein
- R₂: is alkyl, alkenyl, alkynyl, haloalkyl, cycloalkyl, aryl, arylalkyl or cycloalkylalkyl;
- R₃: is -C≡N, -NO₂, 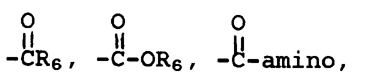 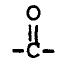 substituted amino, -CF₃ or 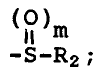
- R₄ and R₅: are each independently selected from R₃, hydrogen, alkyl, alkenyl, alkynyl, haloalkyl, halo, alkoxy, -NHalkyl, -N-(alkyl)₂, -S-alkyl, -O-arylalkyl, -S-arylalkyl or -S-aryl, -O-aryl, -NHarylalkyl;
- R₆: is hydrogen or R₂ and
- m: is the integer 1 or 2.
In addition, potassium channel activators which are pyranyl cyanoguanidine derivatives such as those disclosed in U.S. Patent Application, Serial No. 661,763 (Attorney Docket No. HA488c; filed February 27, 1991, which is a continuation-in-part application of Serial No. 506,632, filed April 9, 1990, the disclosures of which are incorporated by reference herein) may be used in accordance with this invention. Pyranyl cyanoguanidine derivatives have the formula 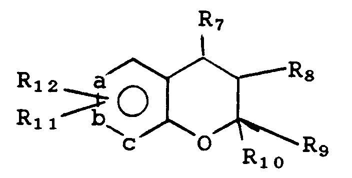
or a pharmaceutically acceptable salt thereof, wherein a, b, and c are all carbons or one of a, b and c can be nitrogen or -NO- and the others are carbons;
- R₇: is 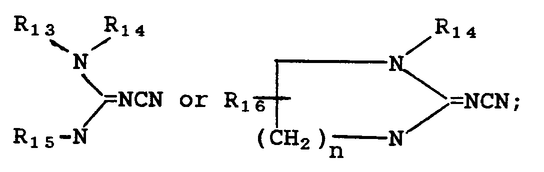
- R₈: is hydrogen, hydroxy, or 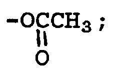
- R₉ and R₁₀: are each independently hydrogen, alkyl or arylalkyl, or, R₉ and R₁₀ taken together with the carbon atom to which they are attached form a 5- to 7-membered carbocyclic ring;
- R₁₁: is selected from hydrogen, alkyl, haloalkyl, alkenyl, alkynyl, cycloalkyl, arylalkyl, cycloalkylalkyl, -CN, -NO₂, -COR₁₇, -COOR₁₇, -CONHR₁₇, -CON(R₁₇)₂, -CF₃, -S-alkyl, -SOalkyl, -SO₂alkyl, 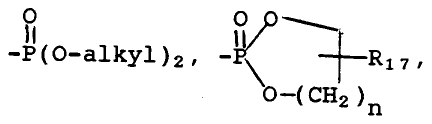 halogen, amino, substituted amino, -O-alkyl, -OCF₃, -OCH₂CF₃, -OCOalkyl, -OCONR₁₇alkyl, -NR₁₇COalkyl and -NR₁₇COOalkyl, -NR₁₇CON(R₁₇)₂ wherein R₁₇ in each of the above groups can be hydrogen, alkyl, haloalkyl, aryl, arylalkyl, cycloalkyl, or (cycloalkyl)alkyl;
- R₁₂: is selected from hydrogen, alkyl, -OH, -O-alkyl, amino, substituted amino, -NHCOR₁₇ (wherein R₁₇ is as defined above), -CN, and -NO₂;
- R₁₃ and R₁₄: are each independently selected from hydrogen, alkyl, alkenyl, aryl, (heterocyclo)alkyl, heterocyclo, arylalkyl, cycloalkyl and (cycloalkyl)alkyl, substituted alkyl wherein the substituents include alkoxy, alkylthio and substituted amino, or R₁₃ and R₁₄ taken together with the nitrogen atom to which they are attached form 1-pyrrolidinyl, 1-piperidinyl, 1-azepinyl, 4-morpholinyl, 4-thiamorpholinyl, 1-piperazinyl, 4-alkyl-1-piperazinyl or 4-arylalkyl-1-piperazinyl, wherein each of the so-formed groups can be substituted with alkyl, alkoxy, alkylthio, halogen or trifluoromethyl;
- R₁₅ and R₁₆: are selected from hydrogen, alkyl, alkenyl, aryl, arylalkyl, cycloalkyl or cycloalkylalkyl; or R₁₆ can be an aryl group fused to 2 carbon atoms of the cyanoguanidine ring portion; and
- n: is an integer selected from 1 to 3.

A preferred such pyranyl cyanoguanidine derivative has the structure: 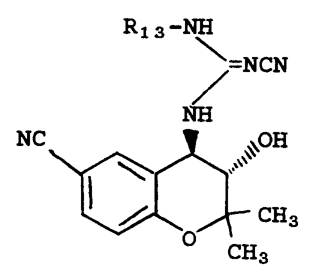
where R₁₃ is phenyl or substituted phenyl, with substituted phenyl (especially 4-chlorophenyl) being preferred.

Examples of other potassium channel activators suitable for use herein include compounds disclosed in pending U.S. Patent Application, Serial No.502,967 (Attorney Docket No. HA531; filed April 2, 1990 by K. Atwal, the disclosure of which is hereby incorporated by reference) having the general formula 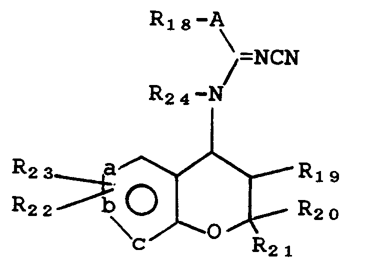
where A is oxygen or sulfur;
- R₁₈: is selected from aryl, arylalkyl, cycloalkyl and (cycloalkyl)alkyl;
- R₁₉: is hydrogen, hydroxy, or 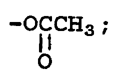
- R₂₀ and R₂₁: are each independently hydrogen, alkyl or arylalkyl, or, R₂₀ and R₂₁ taken together with the carbon atom to which they are attached form a 5- to 7-membered carbocyclic ring;
- R₂₂: is selected from hydrogen, alkyl, haloalkyl, alkenyl, alkynyl, cycloalkyl, arylalkyl, cycloalkylalkyl, -CN, -NO₂, -COR₂₅ -COOR₂₅, -CONHR₂₅, -CON(R₂₅)₂, -CF₃, -S-alkyl, -SOalkyl, -SO₂alkyl, 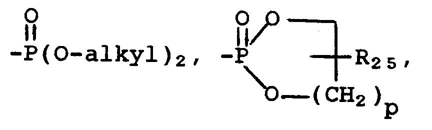 halogen, amino, substituted amino, -O-alkyl, -OCF₃, -OCH₂CF₃, -OCOalkyl, -OCONR₂₅alkyl, -NR₂₅COalkyl and -NR₂₅COOalkyl, -NR₂₅CON(R₂₅)₂, wherein R₂₅ in each of the above groups can be hydrogen, alkyl, aryl, arylalkyl, cycloalkyl, or (cycloalkyl)alkyl;
- R₂₃: is selected from hydrogen, alkyl, -OH, -O-alkyl, amino, substituted amino, -CN, and -NO₂;
- R₂₄: is selected from hydrogen, alkyl, alkenyl, aryl, arylalkyl, cycloalkyl or cycloalkylalkyl; and
- p: is an integer selected from 1 to 3.

Another example of potassium channel activators useful in this invention are those disclosed in pending U.S. Patent Application, Serial No. 630,472 (Attorney Docket No. HA530a; filed December 19, 1990, by K. Atwal, the disclosure of which is hereby incorporated by reference) having the general formula 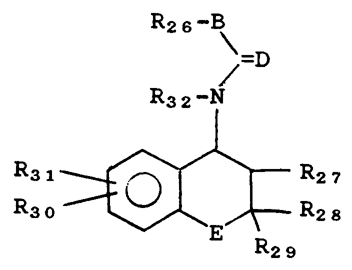
wherein B is NR₃₃-, -O-, -S- or 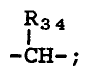
D is oxygen or sulfur; E can be -CH₂-, -O-, NR₃₅-, -S-, -SO-or -SO₂-, where R₃₅ is hydrogen or lower alkyl of 1 to 4 carbons;
- R₂₆: is aryl, arylalkyl, heterocyclo or (heterocyclo)alkyl;
- R₂₇: is hydrogen, hydroxy, or 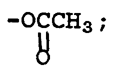
- R₂₈ and R₂₉: are each independently hydrogen, alkyl or arylalkyl, or, R₂₈ and R₂₉ taken together with the carbon atom to which they are attached form a 5- to 7-membered carbocyclic ring;
- R₃₀: is selected from hydrogen, alkyl, haloalkyl, alkenyl, alkynyl, cycloalkyl, arylalkyl, cycloalkylalkyl, -CN, -NO₂, -COR₃₆, -COOR₃₆, -CONHR₃₆, -CONR₂₇, -CF₃, -S-alkyl, -SOalkyl, -SO₂alkyl, 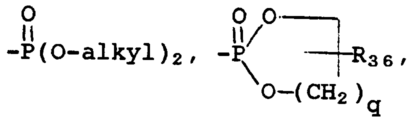 halogen, amino, substituted amino, -O-alkyl, -OCF₃, -OCH₂CF₃, -OCOalkyl, -OCONR₃₆alkyl, -NR₃₆COalkyl, -NR₃₆COOalkyl and -NR₃₆CONR₂ wherein R₃₆ in each of the above groups can be hydrogen, alkyl, aryl, arylalkyl, cycloalkyl, (cycloalkyl)alkyl or haloalkyl;
- R₃₁: is selected from hydrogen, alkyl, halo, -OH, -O-alkyl, amino and substituted amino, -O-alkyl, -OCOalkyl, -OCONR₃₆alkyl, -NRCO₃₆alkyl and -NR₃₆COOalkyl, -NR₃₆CON(R₃₆)₂ wherein R₃₆ in each of the above groups can be hydrogen, alkyl, aryl, arylalkyl, cycloalkyl, (cycloalkyl)alkyl or haloalkyl;
- R₃₂ and R₃₃: are each independently selected from hydrogen, alkyl, arylalkyl; or R₂₆ and R₃₃, or R₂₆ and R₃₂, or R₃₂ and R₃₃ taken together can form a 5- to 7-membered saturated or unsaturated ring, which may further include an aryl group fused to 2 carbon atoms of such 5- to 7-membered ring;
- R₃₄: is hydrogen, hydroxy, alkyl or -O-alkyl; with the proviso that when B is -NH, R₂₇ is hydroxy, R₂₈ and R₂₉ are each methyl, R₃₀ is hydrogen, R₃₁ is 6-cyano, R₃₂ is hydrogen, A and D are each oxygen and R₂₆ must be other than phenyl; and
- q: is an integer selected from 1 to 3.

Additional potassium channel activators useful in this invention are disclosed in pending U.S. Patent Application, Serial No. 618,357 (Attorney Docket No. HA565; filed November 26, 1990, by K. Atwal, the disclosure of which is hereby incorporated by reference) having the general formula 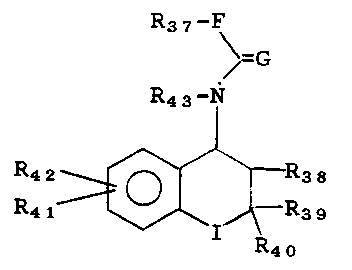
wherein F is -NR₄₄-, -O-, -S- or 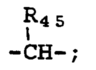
G can be -O-, -S- or -NCN-; provided that when G is -O-or -S-, then I is a single bond; or when G is -NCN-, then I can be a single bond, -CH₂-, -NR₄₆-, -S-, -SO- or -SO₂-, where R₄₆ is hydrogen or lower alkyl of 1 to 4 carbons;
- R₃₇: is aryl, arylalkyl, heterocyclo or (heterocyclo)alkyl;
- R₃₈: is hydrogen, hydroxy, or 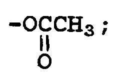
- R₃₉ and R₄₀: are each independently hydrogen, alkyl or arylalkyl, or, R₃₉ and R₄₀ taken together with the carbon atom to which they are attached form a 5- to 7-membered carbocyclic ring;
- R₄₁: is selected from hydrogen, alkyl, haloalkyl, alkenyl, alkynyl, cycloalkyl, arylalkyl, cycloalkylalkyl, -CN, -NO₂, -COR₄₇, -COOR₄₇, -CONHR₄₇, -CON(R₄₇)₂, -CF₃, -S-alkyl, -SOalkyl, -SO₂alkyl, 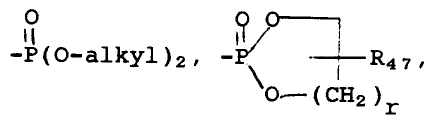 halogen, -OCF₃, -OCH₂CF₃, wherein R₄₇ in each of the above groups can be hydrogen, alkyl, aryl, arylalkyl, cycloalkyl, (cycloalkyl)alkyl or haloalkyl;
- R₄₂: is selected from H, alkyl, halo, -OH, -O-alkyl, amino and substituted amino, -O-alkyl, -OCOalkyl, -OCONRalkyl, -NRCOalkyl, -NRCOOalkyl and -NRCON(R)₂ wherein R in each of the above groups can be hydrogen, alkyl, aryl, arylalkyl, cycloalkyl, (cycloalkyl)alkyl or haloalkyl;
- R₄₃ and R₄₄: are each independently selected from hydrogen, alkyl, arylalkyl; or R₃₇ and R₄₄, or R₃₇ and R₄₃, or R₄₃ and R₄₄ taken together can form a 5- to 7-membered ring, which may further include an aryl group fused to 2 carbon atoms of such 5- to 7-membered ring; R₄₅ is hydrogen, hydroxy, alkyl or -O-alkyl; and
- r: is an integer selected from 1 to 3.

Other potassium channel activators useful in this invention are disclosed in pending U.S. Patent Application Serial No. 618,277 (Attorney Docket No. HA566; filed November 26, 1990, by K. Atwal, the disclosure of which is hereby incorporated by reference) having the general formula 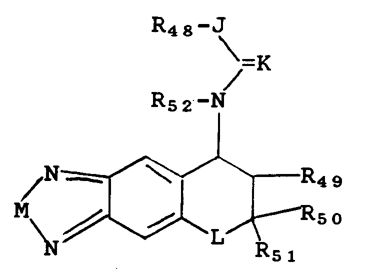
and pharmaceutically acceptable salts thereof, wherein J is -NR₅₃-, -O-, -S- or 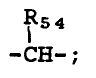
- K: is -O-, -S- or -N-C≡N;
- L: is -O-, 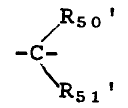 or a single bond;
- M: is -O- or -NH-;
- R₄₈: is aryl, arylalkyl, heterocyclo or (heterocyclo)alkyl;
- R₄₉: is hydrogen, hydroxy or 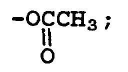
- R₅₀, R₅₀', R₅₁ and R₅₁': are independently selected form hydrogen, alkyl or arylalkyl, or, R₅₀ and R₅₁ (or R₅₀' and R₅₁') taken together with the carbon atom to which they are attached form a 5- to 7-membered ring; with the proviso that if R₅₀ and/or R₅₁ are other than hydrogen, then R₅₀' and R₅₁' are each hydrogen;
- R₅₂ and R₅₃: are each independently hydrogen, alkyl or arylalkyl; or R₄₈ and R₅₂, or, R₅₂ and R₅₃ or R₄₈ and R₅₃ taken together can form a 5- to 7-membered ring, which may further include an aryl group fused to 2 carbon atoms of such 5- to 7-membered ring; and
- R₅₄: is hydrogen, hydroxy, alkyl or -O-alkyl.

Additional compounds useful in accordance with this invention are disclosed in pending U.S. Patent Application, Serial No. 685,323 (Attorney Docket No. HA577; filed April 15, 1991, by K. Atwal, the disclosure of which is hereby incorporated by reference), having the general formula 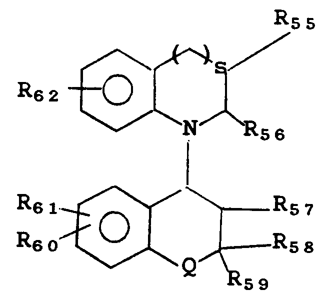
wherein Q is -O-, 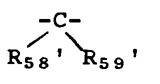
or a single bond;
- R₅₅ and R₅₆: are independently selected from hydrogen, alkyl, haloalkyl, cycloalkyl, arylalkyl, -CN, -NO₂, -COR₆₃, -COOR₆₃, -CONHR₆₃, -CON(R₆₃)₂, halo wherein R₆₃ in each of the above groups can be hydrogen, alkyl, haloalkyl, aryl, arylalkyl, cycloalkyl, or (cycloalkyl)alkyl;
- R₅₇: is hydrogen, hydroxy, or 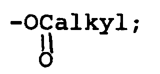
- R₅₈, R₅₈', R₅₉ and R₅₉': are each independently selected from hydrogen, alkyl or arylalkyl, or, R₅₈ and R₅₉ (or R₅₈' and R₅₉') taken together with the carbon atom to which they are attached form a 5- to 7-membered ring; with the proviso that if R₅₈ and/or R₅₉ are other than hydrogen, then R₅₈' and R₅₉' are each hydrogen;
- R₆₀: is hydrogen, alkyl, haloalkyl, alkenyl, alkynyl, cycloalkyl, arylalkyl, (cycloalkyl)alkyl, -CN, -NO₂, -COR₆₃, -COOR₆₃, -CONHR₆₃, -CON(R₆₃)₂, -CF₃, -S-alkyl, -SOalkyl, 1-SO₂alkyl, 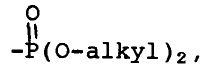 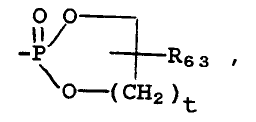 halogen, amino, substituted amino, -O-alkyl, -OCF₃, -OCH₂CF₃, -OCOalkyl, -OCONR₆₃alkyl, -NR₆₃COalkyl, -NR₆₃COOalkyl and -NR₆₃CON(R₆₃)₂ wherein R₆₃ is as defined previously;
- R₆₁: is selected from hydrogen, alkyl, -OH, -O-alkyl, amino, substituted amino, -NHCOR₆₃, -CN, and -NO₂ wherein R₆₃ is as defined previously;
- R₆₂: is hydrogen, alkyl, haloalkyl, cycloalkyl, -O-R₆₃, -CN, -NO₂, -CF₃, halo, -S-alkyl, -COR₆₃, -COOR₆₃, -NR₆₃COalkyl and -OCOalkyl wherein R₆₃ is as defined previously;
- s: is 0 or the integer 1; and
- t: is an integer of 1 to 3.

Any pharmaceutically acceptable compound containing a sulfhydryl moiety is suitable for use in accordance with this invention. Known classes of compounds having a sulfhydryl moiety which are particularly suitable include angiotensin converting enzyme (ACE) inhibitors and anti-inflammatory agents such as N-acetylcysteine and penicillamine. The preferred compounds are ACE inhibitors. The sulfhydryl containing compound may also be administered in prodrug form, e.g. zofenopril (S-benzoyl).

The ACE inhibitors which may be employed herein preferably include any of those disclosed in U.S. Patent No. 4,046,889 to Ondetti et al. mentioned above, with captopril, that is, 1-[(2S)-3-mercapto-2-methylpropionyl]-L-proline, being preferred, and mercaptoacyl derivatives of substituted prolines such as any of those disclosed in U.S. Patent No. 4,316,906 with zofenopril being preferred.

Other examples of mercapto containing ACE inhibitors that may be employed herein include rentiapril (fentiapril, Santen) disclosed in Clin. Exp. Pharmacol. Physiol. 10:131 (1983); as well as pivopril, that is 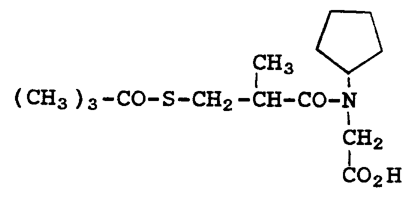
and YS980, that is

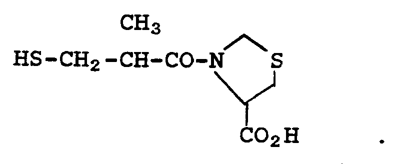

The disclosures of the above-mentioned patents, patent applications and other references are incorporated herein by reference.

It is also within the scope of this invention that the sulfhydryl containing compounds themselves may be used alone as potassium channel activators.

In carrying out the method of the present invention, potassium channel openers and pharmaceutically acceptable sulfhydryl moiety containing compounds may be co-administered to mammalian species, such as monkeys, dogs, cats, rats, humans, etc. and as such may be independently incorporated in a conventional systemic dosage form, such as a tablet, capsule, elixir and injectable or combined in a conventional systemic dosage form. The above dosage forms will also include the necessary carrier material, excipient, lubricant, buffer, antibacterial, bulking agent (such as mannitol), anti-oxidants (ascorbic acid or sodium bisulfite) or the like. Oral dosage forms are preferred, although parenteral forms are quite satisfactory as well.

The dose administered must be carefully adjusted according to age, weight and condition of the patient, as well as the route of administration, dosage form and regimen and the desired result.

Thus for oral administration, a satisfactory result may be obtained employing the potassium channel opener in an amount from about 0.01 mg/kg to about 100 mg/kg and preferably from about 1 mg/kg to about 10 mg/kg. The dose of the sulfhydryl moiety containing compound will necessarily depend upon the compound which provides the sulfhydryl moiety. For example, when the sulfhydryl moiety is provided by an angiotensin converting enzyme inhibitor, satisfactory results may be obtained by employing the ACE inhibitor in an amount within the range of from about 0.01 mg/kg to about 100 mg/kg and preferably from about 0.1 mg/kg to about 25 mg/kg for oral administration.

The co-administration described above may be accomplished in the dosage forms as described above in single or divided doses of one to four times daily. It may be advisable to start a patient on low doses and work up gradually to high doses.

Tablets of various sizes can be prepared, e.g., of about 5 to 700 mg in total weight, containing the active substance or substances in the ranges described above, with the remainder being a physiologically acceptable carrier of other materials according to accepted pharmaceutical practice. These tablets can, of course, be scored to provide for fractional doses. Gelatin capsules can be similarly formulated.

Liquid formulations can also be prepared by dissolving or suspending the active substance or substances in a conventional liquid vehicle acceptable for pharmaceutical administration so as to provide the desired dosages in one to four teaspoonfuls.

Such dosage forms can be administered to the patient on a regimen of one to four doses per day.

Illustrative of the adjuvants which may be incorporated in tablets are the following: a binder such as gum tragacanth, acacia, corn starch or gelatin; an excipient such as dicalcium phosphate or cellulose; a disintegrating agent such as corn starch, potato starch, alginic acid or the like; a lubricant such as stearic acid or magnesium stearate; a sweetening agent such as sucrose, lactose or saccharin; a flavoring agent such as orange, peppermint, oil of wintergreen or cherry. When the dosage unit form is a capsule, it may contain in addition to materials of the above type a liquid carrier such as a fatty oil. Various other materials may be present as coatings or to otherwise modify the physical form of the dose unit. For instance, tablets or capsules may be coated with shellac, sugar or both. A syrup of elixir may contain the active compound, water, alcohol or the like as the carrier, glycerol as solubilizer, sucrose as sweetening agent, methyl and propyl parabens as preservatives, a dye and a flavoring such as cherry or orange.

According to another modification, in order to more finely regulate the dosage schedule, the active substances may be administered separately in individual dosage units at the same time or carefully coordinated times. Since blood levels are built up and maintained by a regulated schedule of administration, the same result is achieved by the simultaneous presence of the two substances. The respective substances can be individually formulated in separate unit dosage forms as described above.

Fixed combinations of potassium channel openers and pharmaceutically acceptable sulfhydryl moiety containing compounds are more convenient and are preferred, especially in tablet or capsule form for oral administration.

In formulating the compositions, the active substances, in the amounts described above, are compounded according to accepted pharmaceutical practice with a physiologically acceptable vehicle, carrier, excipient, binder, preservative, stabilizer, flavor, etc., in the particular type of unit dosage form.

The active substances described above form commonly known, pharmaceutically acceptable salts such as alkali metal and other common basic salts or acid addition salts, etc. References to these substances are therefore intended to include those common salts known to be substantially equivalent to the present compound.

The formulations as described above will be administered for a prolonged period, that is, for as long as the potential for ischemia remains or the symptoms continue. Sustained release forms of such formulations which may provide such amounts biweekly, weekly, monthly and the like may also be employed. A dosing period of at least one to two weeks are required to achieve minimal benefit.

The following examples and preparations describe the manner and process of making and using the invention and are illustrative rather than limiting. It should be understood that there may be other embodiments which fall within the spirit and scope of the invention as defined by the claims appended hereto.

### Example

The following experiment was carried out to demonstrate the use of the combination of the potassium channel opener cromakalim and the sulfhydryl-containing compound zofenopril.

### Method Male Sprague-Dawley rats (450-550 g) were

anesthetized using 100 mg/kg sodium pentobarbital (i.p.). They were intubated and then treated with i.v. heparin (1000 U/kg). While mechanically ventilated, hearts were perfused *in situ* via retrograde cannulation of the aorta. The hearts were then excised and quickly moved to a Langendorff apparatus where they were perfused with Krebs-Henseleit bicarbonate buffer (112 mM sodium chloride, 25 mM sodium bicarbonate, 5 mM potassium chloride, 1.2 mM magnesium sulfate, 1 mM potassium diphosphate, 1.25 mM calcium chloride, 11.5 mM glucose and 2mM pyruvate bubbled with 95% oxygen - 5% carbon dioxide) at a constant perfusion pressure (75 mm Hg). A water-filled latex balloon, attached to a metal cannula, was inserted into the left ventricle and connected to a Statham pressure transducer for measurement of left ventricular pressure. The hearts were allowed to equilibrate for 15 minutes, at which time end diastolic pressure (EDP) was adjusted to 5 mm Hg and the balloon pressure was maintained for the duration of the experiment. Pre-ischemia or pre-drug function, heart rate and coronary flow (extracorporeal electromagnetic flow probe, Carolina Medical Electronics, King, N.C.) were then measured. Cardiac function was determined using double product of heart rate (HR) X left ventricular developed pressure (LVDP) divided by 1000. Cardiac temperature was maintained throughout the experiment by submerging the hearts in a heated chamber filled with 37°C buffer.

All hearts were retreated with vehicle or drug for 10 minutes before the onset of ischemia. All drugs were given only before ischemia. The hearts were subjected to 25 minutes of global ischemia and 30 minutes of reperfusion. At the end of the experiment, EDP and LDH release were measured and used as an index of severity of ischemia.

### Results

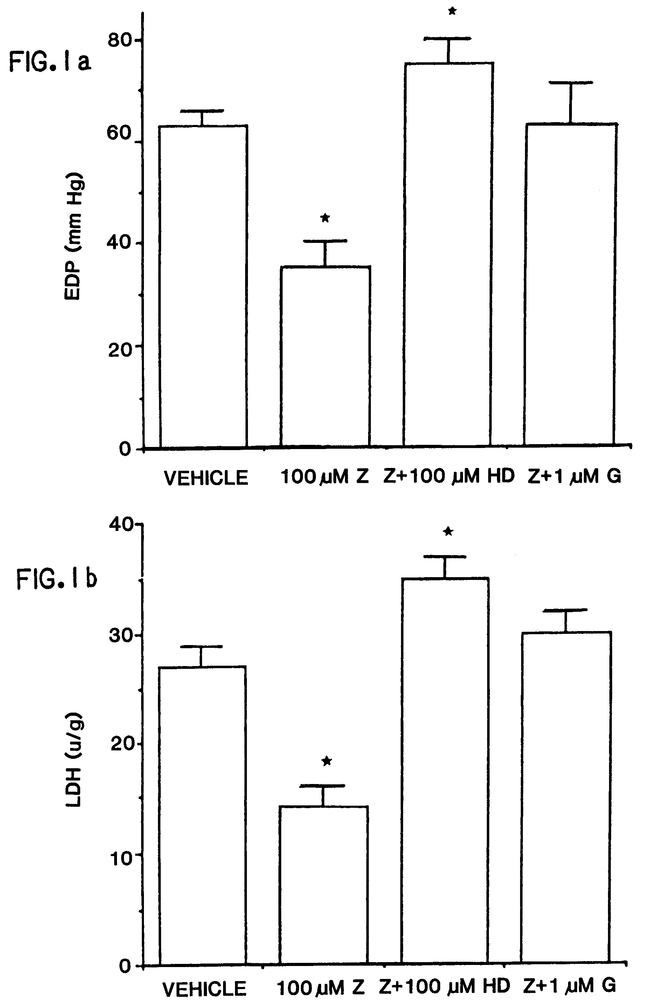

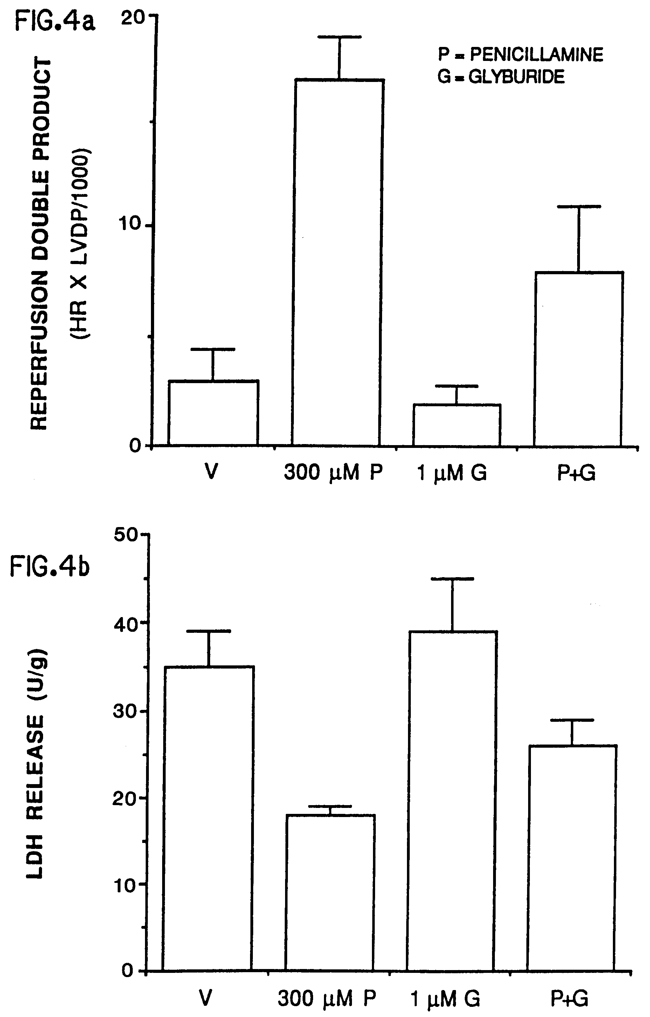

Figures 1a and 1b to 4a and 4b have been included to show that the SH-containing compound is acting via activation of potassium channels.

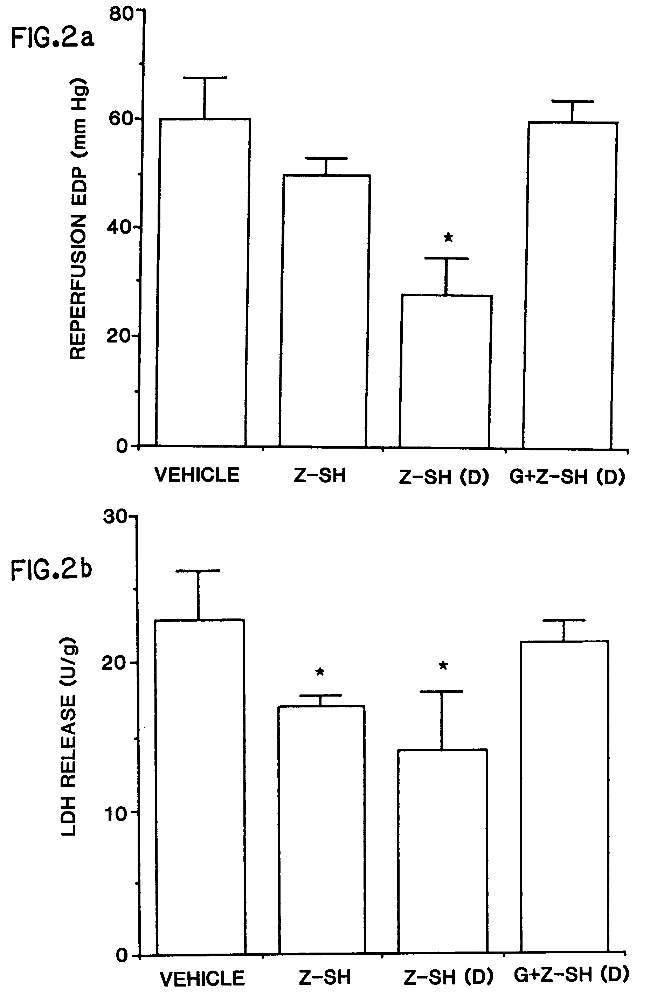

As shown in Figures 1a and 1b, zofenopril prodrug significantly reduced the severity of myocardial ischemia as measured by EDP and LDH release. Increases in EDP during ischemia is due to a decreased cardiac compliance. LDH release is used as an index of cell viability. LDH levels increase during cardiac infarction. Therefore, decreases in EDP and LDH indicate reduced severity of ischemia. Glyburide and sodium -5- hydroxy-decanoate are known ATP-sensitive potassium channel blockers. When these compounds are given with zofenopril prodrug the levels of EDP and LDH increase, as seen in Figures 1a and 1b. In addition as shown in Figure 2a and 2b, glyburide also reverses the cardioprotective effect of the free-SH form of zofenopril. These data indicate that both forms of zofenopril are protecting ischemic hearts via the activation of ATP-sensitive potassium channels in the heart.

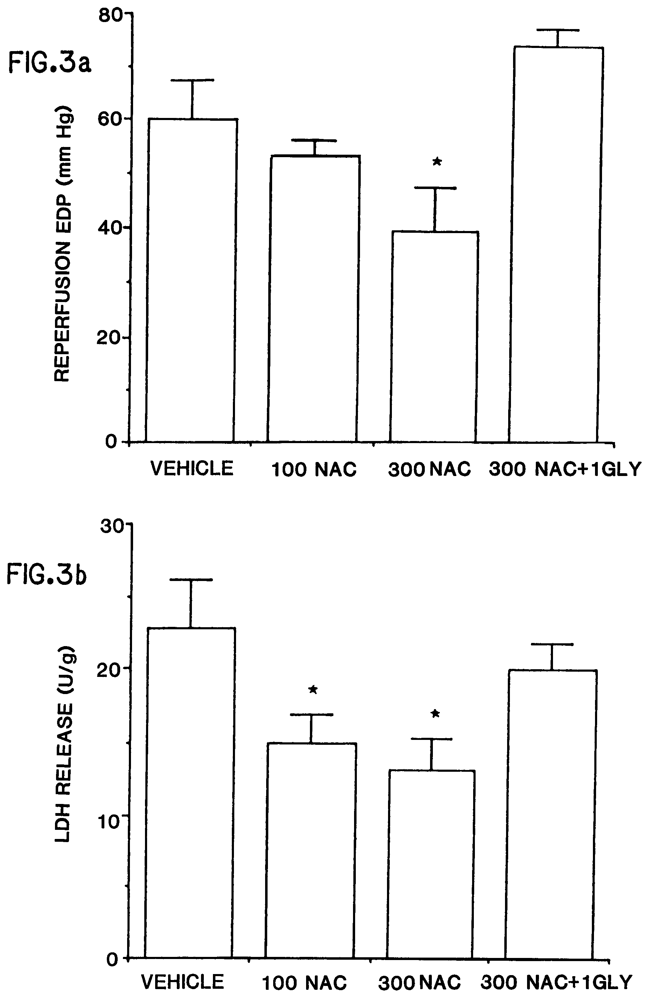

As shown in figures 3a and 3b, the SH-containing anti-inflammatory compound N-acetyl-cysteine protected ischemic cardiac tissue and this effect was also reversed by glyburide. Figures 4a and 4b show the SH-containing compound penicillamine also protects cardiac tissue. Penicillamine significantly improved reperfusion double product and significantly reduced LDH release. These effects were partially reversed by glyburide (G).

Therefore it has been shown that SH-containing compounds themselves are potassium channel activators.

The effect of the combination of a potassium channel activator and a sulfhydryl containing compound was determined using a technique known as isobolographic analysis (R. J. Tallarida et al, "Minireview-Satistical Analysis of Drug-Drug and Site-Site Interactions with Isobolograms", Life Sciences, 45:947-961, 1989). This technique is commonly used to investigate the interaction between drugs. The isobolographic analysis indicated that all the ratios tested have superadditive interaction.

Several specific concentration ratios (3:1, 12:1 and 30:1) of cromakalim to zofenopril respectively were tested and the effects of the combination determined. The concentration response curves for cromakalim and zofenopril were expressed as percentage increase in time to contracture from control. Time to contracture was measured as the time during ischemia for end diastolic pressure to increase by 5 mm Hg.

EC₅₀ for percentage change in time to contracture for cromakalim and zofenopril given alone or in the concentration ratios indicated were calculated.

| Cromakalim | Zofenopril | 3:1 | | 12:1 | | 30:1 | |
|---|---|---|---|---|---|---|---|
| | | Crom | Zof | Crom | Zof | Crom | Zof |
| 39.7 | 75 | 5.15 | 0.128 | 5.96 | 0.48 | 9.3 | 0.29 |
| All values given in µM | | | | | | | |

As can be seen from the above table, the EC₅₀ value for cromakalim alone is 39.7µM. However, when given in combination with zofenopril (3:1, 12:1 and 30:1), the EC₅₀ value was significantly lower which indicates increased anti-ischemic potency; i.e., the lower the dose required to give a 50% increase (EC₅₀) in time to contracture. Furthermore, when this data is plotted on an isobologram (below), the graph clearly indicates superadditivity (at concentration ratios between 3:1 to 30:1 (cromakalim:zofenopril)), as each point clearly lies to the left of the line of theoretical activity. 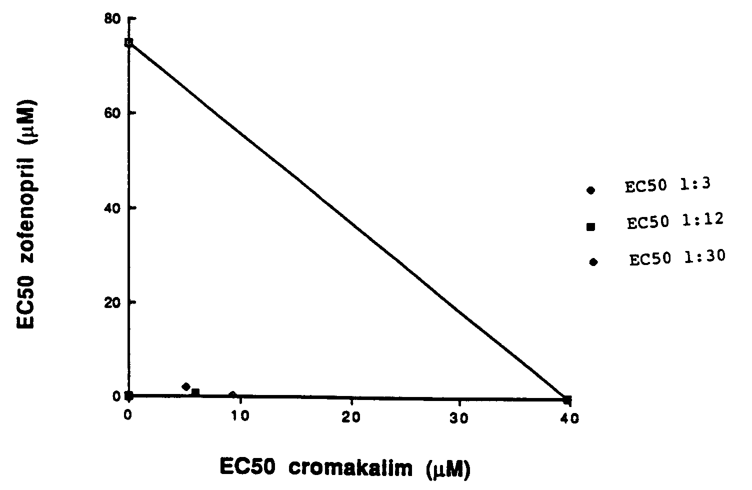
In view of the results shown above, it may be concluded that SH-containing compounds themselves are useful as potassium channel activators. Furthermore, the ability of sulfhydryl containing compounds to potentiate the anti-ischemic activity of potassium channel openers would not be expected. If sulfhydryl containing compounds and potassium channel openers were thought to be anti-ischemic via the same mode of potassium activation, they would be merely additive in their protective effects. It has been found however that their PCA effects are superadditive i.e. the combination of a SH-containing compound and a potassium channel opener gives a surprising superadditive anti-ischemic effect.

## Claims

1. Use of a potassium channel opener and a pharmaceutically acceptable compound having a sulfhydryl moiety for manufacturing a medicament in separate or combined dosage forms for treating ischemia and/or reducing myocardial infarct size in a mammalian specie.

2. The method as recited in Claim 1 wherein the ratio of potassium channel opener and a pharmaceutically acceptable compound having a sulfhydryl moiety is between about 1:1 to about 100:1.

3. The method as recited in Claim 1 wherein the ratio of potassium channel opener and a pharmaceutically acceptable compound having a sulfhydryl moiety is between about 3:1 to about 30:1.

4. The method as recited in Claim 1 wherein the potassium channel opener is cromakalim.

5. The method as recited in Claim 1 wherein the sulfhydryl moiety is provided by an angiotensin converting enzyme inhibitor.

6. The method as recited in Claim 5 wherein the angiotensin converting enzyme inhibitor is captopril.

7. Use as recited in Claim 5 wherein the angiotensin converting enzyme inhibitor is zofenopril.

8. Use as recited in Claim 1 wherein the sulfhydryl moiety is provided by an anti-inflamatory compound.

9. Use as recited in Claim 8 wherein the anti-inflammatory compound is N-acetylcysteine.

10. Use as recited in Claim 8 wherein the anti-inflammatory compound is penicillamine.

11. Use as recited in Claim 5 wherein the potassium channel opener is for administration in an amount from about 0.01 mg/kg to about 100 mg/kg and the angiotensin converting enzyme inhibitor is for administration in an amount from about 0.01 mg/kg to about 100 mg/kg.

12. Use as recited in Claim 11 wherein the potassium channel opener is for administration in an amount from about 1 mg/kg to about 10 mg/kg and the angiotensin converting enzyme inhibitor is for administration in an amount from about 0.1 mg/kg to about 25 mg/kg.

13. Use as recited in Claim 1 wherein said potassium channel opener in combination with said sulfhydryl moiety comprising compound is for oral or parental administration.
